# EUROPEAN PATENT APPLICATION

(11) **EP 3 401 396 A1**
(43) Date of publication of application: **14.11.2018**
(21) Application number: 18181011.0
(22) Date of filing: 03.10.2013
(51) Int. Cl.: C12N 9/02, C12Q 1/68, A61K 31/00, A61K 31/015, A61K 35/747, C12Q 1/6883, C12Q 1/689, G01N 33/49, G01N 30/00, G06F 19/00, G06F 19/22, G16H 50/30, A61K 35/00

(54) **TREATING OR PREVENTING ATHEROSCLEROSIS OR ASSOCIATED DISEASES BY BETA-CAROTENE**

(30) Priority: 03.10.2012 US 201261709255 P
(62) Divisional of application: 13771514.0
(71) Applicant: Metabogen AB, 411 26 Gothenburg (SE)
(72) Inventor: Backhed, Fredrik, S-429 34 Kullavik (SE); Karlsson, Fredrik H, S-413 26 Gothenburg (SE); Nielsen, Jens, S-41128 Gothenburg (SE)
(74) Representative: Dehns

(57) **Abstract**

The present invention provides the use of beta-carotene in treating or preventing atherosclerosis or atherosclerotic associated disease, wherein the beta-carotene is a probiotic bacteria producing beta-carotene such as Lactobacillus reuteri.

## Description

### FIELD OF THE INVENTION

The present invention relates generally to medicine. More specifically the invention relates to the identification and prevention of a person having risk for cardiovascular diseases including atherosclerosis and associated diseases by determining the bacterial presence of specific groups or species and also metabolic functions in the person's gut microbiota. The present invention thus relates to the identification of a person having risk for atherosclerosis and associated diseases by the person's gut microbiome and the prevention of such diseases.

### BACKGROUND OF THE INVENTION

Within the body of a healthy adult, microbial cells are estimated to outnumber human cells by a factor of ten to one. These communities, however, remain largely unstudied, leaving almost entirely unknown their influence upon human development, physiology, immunity, nutrition and health.

Traditional microbiology has focused on the study of individual species as isolated units. However many, if not most, have never been successfully isolated as viable specimens for analysis, presumably because their growth is dependent upon a specific microenvironment that has not been, or cannot be, reproduced experimentally. Among those species that have been isolated, analyses of genetic makeup, gene expression patterns, and metabolic physiologies have rarely extended to inter-species interactions or microbe-host interactions. Advances in DNA sequencing technologies have created a new field of research, called metagenomics, allowing comprehensive examination of microbial communities, even those comprised of uncultivable organisms. Instead of examining the genome of an individual bacterial strain that has been grown in a laboratory, the metagenomic approach allows analysis of genetic material derived from complete microbial communities harvested from natural environments. For example, the gut microbiota complements our own genome with metabolic functions that affects human metabolism and may thus play an important role in health and disease.

Atherosclerotic disease, with manifestations such as myocardial infarction and stroke, is the major cause of severe disease and death among subjects with the metabolic syndrome. The disease is believed to be caused by accumulation of cholesterol and recruitment of macrophages to the arterial wall and can thus be considered both as a metabolic and inflammatory disease. Since the first half of the 19^{th} century infections have been suggested to cause or promote atherosclerosis by augmenting pro-atheroslerotic changes in vascular cells.

However there is still a need for better ways to early identify a person having risk for cardiovascular diseases including atherosclerosis and associated diseases.

### SUMMARY OF THE INVENTION

The invention herein demonstrates how metagenomics can be used to identify not only specific species in the microbiota but also identify enriched metabolic functions in the gut microbiota. Using shotgun sequencing of the gut metagenome the genus *Collinsella* was enriched in patients with stenotic atherosclerosclerotic plaques in the carotid artery leading to cerebrovascular events (i.e. symptomatic atherosclerosis), while *Roseburia* and *Eubacterium* were enriched in healthy controls. This information makes it possible to design possible prevention strategies for correcting the microbiota in people in a risk group of contracting disease. We also found that the metagenomes of control subjects were predominantly associated with the 'Bacteroides' enterotype while the patients were associated with the 'Ruminococcus' enterotype. Using the invention herein and mapping the metagenomes onto metabolic maps we can obtain further characterization of the functional capacity, and this reveals that the patients' metagenomes are enriched in genes encoding for peptidoglycan synthesis and depleted in phytoene dehydrogenase. The identification of phytoene dehydrogenase as the most significantly different gene between patients and controls led us to analyze the serum levels of β-carotene, and we found that patients have reduced levels of this anti-oxidant. The later also led us to design a prevention strategy of distributing β-carotene or β-carotene - producing probiotic bacteria to people at risk. The invention thus illustrates that metagenomics can be used to identify metabolic functions of the gut microbiota that are associated with metabolites and markers in serum that may influence atherosclerosis prevention, onset and development. The invention can thus be used to identify serum markers (as well as gut microbiota markers) that have a role in atherosclerosis and associated diseases.

Thus, the present invention relates to cardiovascular disease, in particular atherosclerosis, and to the use of metagenomics to identify a compositional or functional alteration of the gut metagenome related to atherosclerosis or atherosclerotic associated disease. In preferred embodiments whole genome metagenomics is used. In more preferred embodiments the use of the invention comprises comparing the gut metagenome of subjects with atherosclerosis or atherosclerotic associated disease to the gut metagenome of control subjects and identifying differences between the metagenome of the disease subjects and the control subjects in the type or amount of microorganisms or the type or amount of genes which are present. The invention thus further relates to diagnostic and therapeutic methods based on an analysis of a subject's gut metagenome.

Thus, further aspects of the present invention provide a method for determining if a subject is in a risk group for developing atherosclerosis or atherosclerotic associated disease, or if a subject has atherosclerosis or atherosclerotic associated disease, said method comprising analysing the gut flora of said subject for the presence of specific bacterial groups or species. In preferred embodiments the bacteria analysed is of the genus *Collinsella, Roseburia* or *Eubacterium.*

In other preferred aspects the present invention provides a method for determining if a subject is in a risk group for developing atherosclerosis or atherosclerotic associated disease, or if a subject has atherosclerosis or atherosclerotic associated disease, said method comprising analysing the gut flora of said subject for the presence of one or more phytoene dehydrogenase genes.

In other preferred aspects the present invention provides a method for determining if a subject is in a risk group for developing atherosclerosis or atherosclerotic associated disease, or if a subject has atherosclerosis or atherosclerotic associated disease, said method comprising analysing the levels of β-carotene in the serum of said subject.

In other preferred aspects the present invention provides a method for determining if a subject is in a risk group for developing atherosclerosis or atherosclerotic associated disease, or if a subject has atherosclerosis or atherosclerotic associated disease, said method comprising analysing the gut flora of said subject for the presence of one or more peptidoglycan genes.

In other aspects, the present invention provides a method of treating or preventing atherosclerosis or atherosclerotic associated disease in a subject having or at risk of having atherosclerosis or atherosclerotic associated disease, comprising the administration of an effective amount of β-carotene. Other embodiments provide a method of treating or preventing atherosclerosis or atherosclerotic associated disease in a subject having or at risk of having atherosclerosis or atherosclerotic associated disease, comprising the administration of an effective amount of an antimicrobial agent or vaccine, wherein said agent or vaccine has the effect of reducing levels of bacteria which are elevated in the gut flora of the subject compared to the level in a control subject.

In all the aspects of the invention a preferred type of cardiovascular disease is atherosclerosis or atherosclerotic (atherosclerosis) associated diseases (which can also be referred to as atherosclerotic or atherosclerosis related diseases), preferably atherosclerosis, although the methods of the invention can apply to other types of cardiovascular disease.

Atherosclerosis is a chronic disease that can remain asymptomatic for many years. Atherosclerotic plaques are separated into stable and unstable plaques. There are several atherosclerotic associated diseases depending on the arteries that are affected and include but are not limited to coronary heart disease, carotid artery disease, chronic kidney disease and peripheral arterial disease.

### BRIEF DESCRIPTION OF THE FIGURES AND TABLES

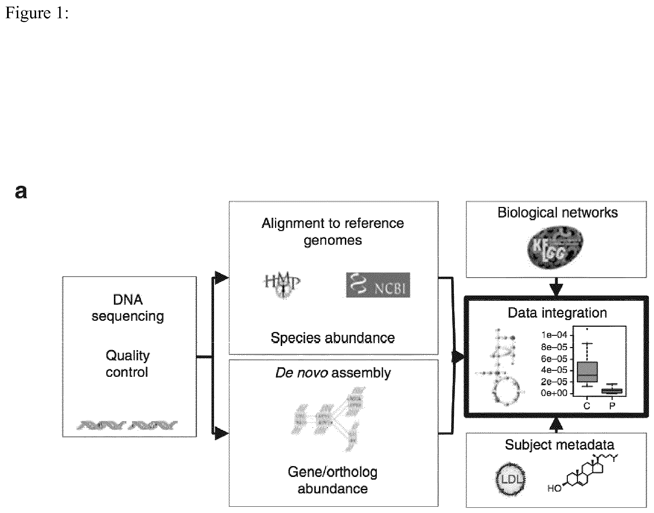

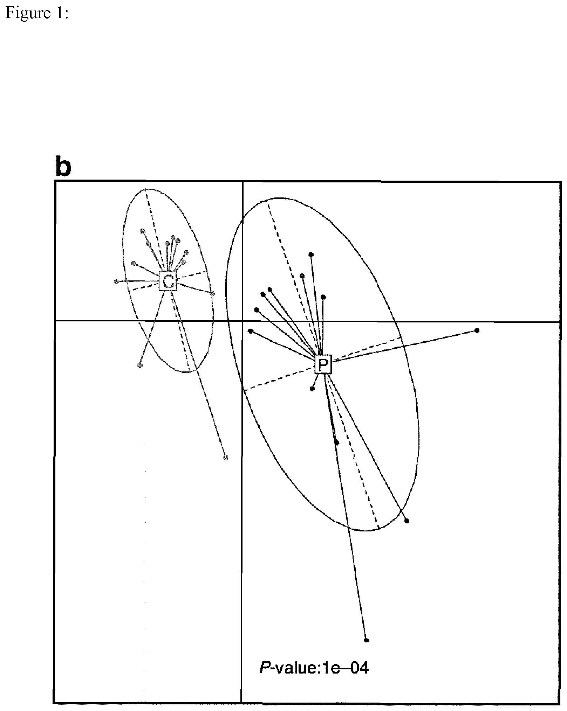

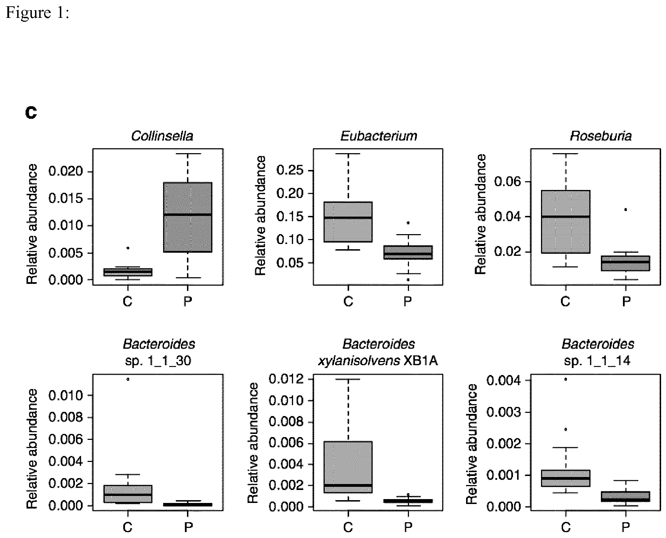

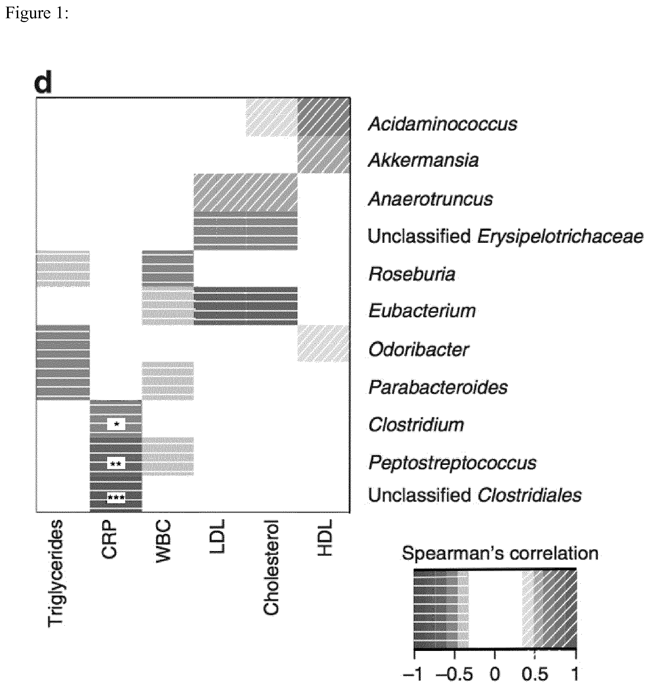

**Figure 1****.** Microbial composition differs between patients with symptomatic atherosclerosis and healthy controls, and correlates with atherosclerotic biomarkers.
   a) Illustration of our bioinformatics pipeline MEDUSA for analyzing metagenome data to elucidate its relation to human metabolic disease. Sequence reads from the gut metagenome were generated with high-throughput sequencing technology and subjected to quality control. High-quality reads were used for alignment to reference genomes to estimate species abundance. *De novo* assembly of the metagenome allows for discovery of new genes not yet found in databases. Annotation of genes to KEGG allows for integration of information at the gene level with the metabolic network. Data on plasma metabolites and proteins together with gut metagenomic data constitute a basis for discovery of mechanisms for gut metagenome association with etiology of complex diseases.
   b) Principal component analysis of microbial species abundance using health status as instrumental variable. P is patients (n=12), C is controls (n=13). The relation between microbial abundance and health status was assessed with Monte Carlo simulations with 10,000 replications by which a P value was calculated.
   c) Abundance of bacterial genera and species that differ between patients (n=12) with symptomatic atherosclerosis (P) and controls (n=13) (C). Adj. P<0.05 for all.
   d) Bacterial genera correlating with biomarkers of atherosclerosis, using Spearman's correlation. All samples were used for correlations with triglycerides, C-reactive protein (CRP) (n=27 respectively) and white blood cell count (WBC) (n=23). Only controls were used for LDL, HDL and cholesterol correlations to avoid interactions with possible drug effects (n=15). *Adj. P <0.05, **Adj. P <0.01, ***Adj. P <0.001.

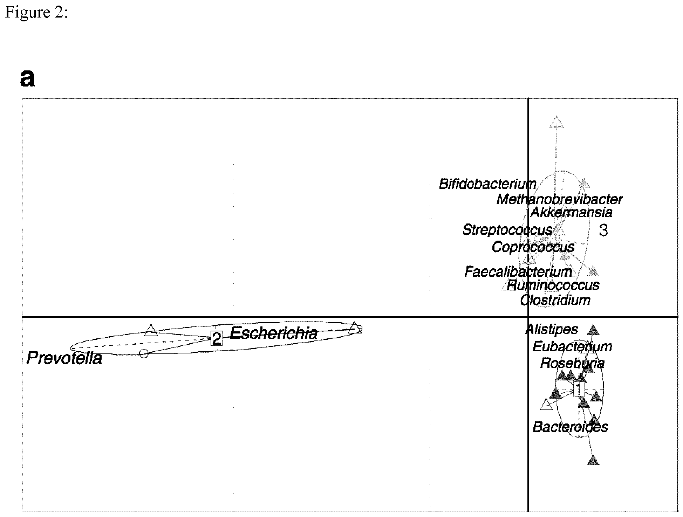

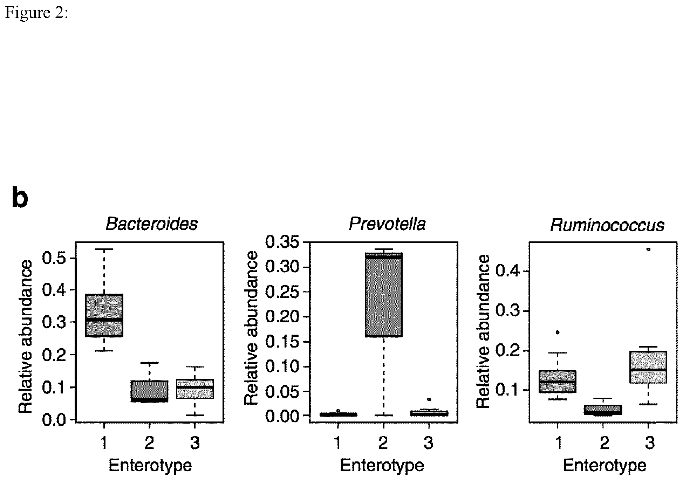

**Figure 2****.** Symptomatic atherosclerosis is related to gut enterotypes.
   a) Three enterotypes in our cohort based on the abundance of genera. Controls and patients are denoted by filled triangles and empty triangles, respectively. Data in circle 1 is enterotype 1, in 2 is enterotype 2 and in 3 is enterotype 3.
   b) Abundance of *Bacteroides, Prevotella* and *Ruminococcus,* proposed drivers of the three enterotypes.

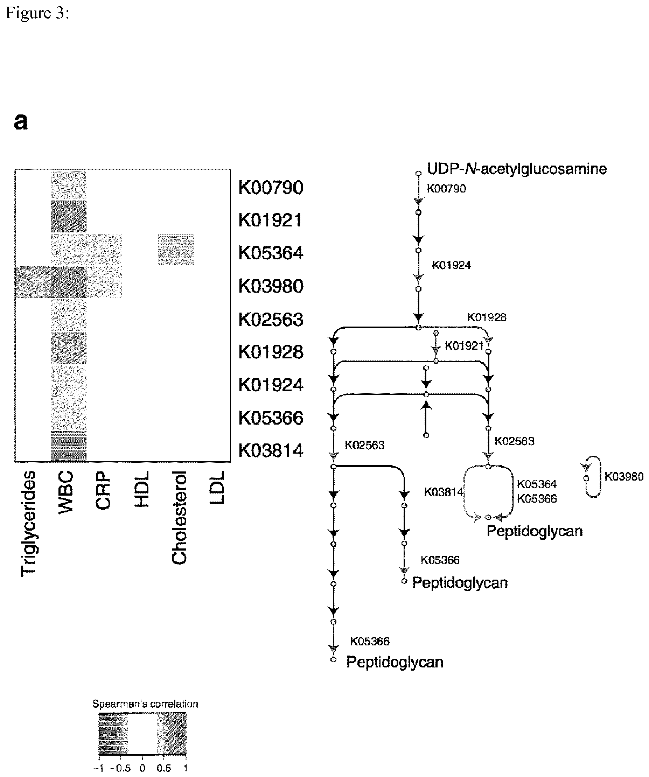

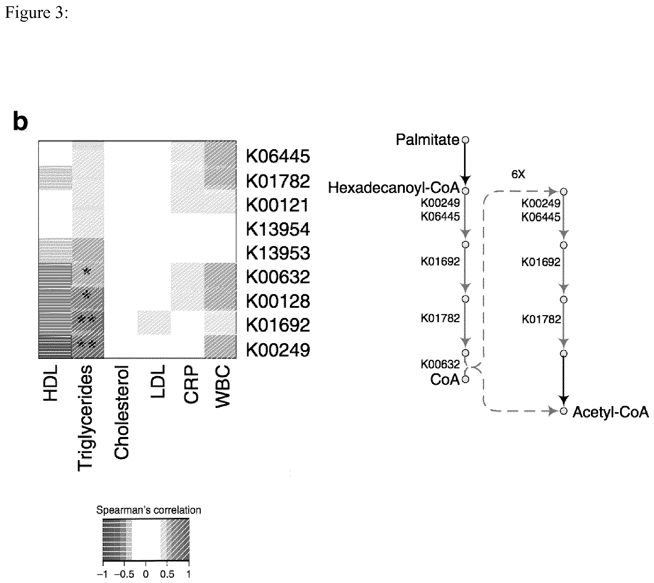

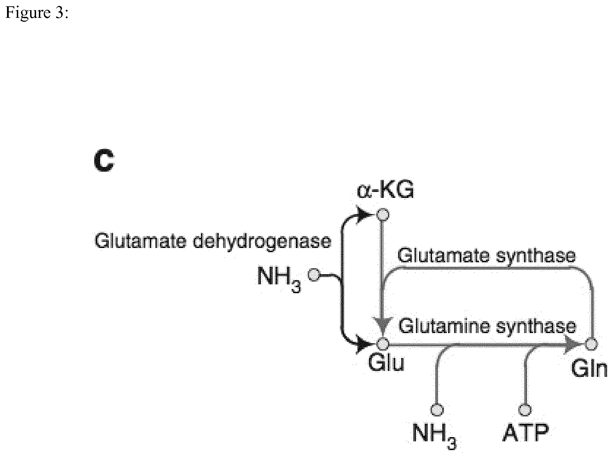

**Figure 3****.** Abundance of KEGG orthologies (KOs) is associated with symptomatic atherosclerosis.
   a) Peptidoglycan KOs were enriched in patients and 8 out of 9 KOs correlated positively with white blood cell levels.
   b) β-oxidation KOs correlates with plasma triglyceride levels.
   c) The GS-GOGAT system (K00265, K00266 and K01915) with high affinity for ammonium is enriched in patients.
   *Adj. P <0.05, **Adj. P <0.01, ***Adj. P <0.001. All samples were used for correlations with triglycerides, C-reactive protein (CRP) (n=27 respectively) and white blood cell count (WBC) (n=23). Only controls were used for LDL, HDL and cholesterol correlations to avoid interactions with possible drug effects (n=15).
**Figure 4****.** Phytoene dehydrogenase, K10027 is enriched in the gut metagenome of healthy controls. β- carotene (P=0.05) but not lycopene (P=0.35) was enriched in serum of healthy controls. (P) is patients, (C) is controls.
**Figure 5** Relative abundance of the six most abundant microbial phyla in the 27 subjects in our cohort.
**Figure 6** Relative abundance of the 30 most abundant microbial genera in the 27 subjects in our cohort
**Figure 7** Relative abundance of the 30 most abundant microbial species in the 27 subjects in our cohort.
**Figure 8** Relative abundance of the 30 most abundant microbial genomes in the 27 subjects in our cohort.

### DETAILED DESCRIPTION OF THE INVENTION AND PREFERRED EMBODIMENTS THEREOF

The gut microbiota has been implicated as an environmental factor influencing adiposity and obesity, by modulating host lipid metabolism. Additionally, the gut microbiota is a source of inflammatory molecules such as lipopolysaccharide and peptidoglycan that may contribute to onset and development of metabolic disease. Atherosclerotic disease, with manifestations such as myocardial infarction and stroke, is characterized by accumulation of cholesterol and recruitment of macrophages to the arterial wall. In a recent study, we used pyrosequencing of the 16S rDNA genes to show that atherosclerotic plaques contain bacterial DNA with phylotypes common to the gut microbiota and that the amount of bacterial DNA in the plaque correlated with inflammation (7). A further indication of strong links between the gut microbiota and atherosclerosis are findings that metabolism of the gut microbiota influences metabolism of phosphatidylcholine in the human body (8).

Many studies of complex microbiota, including the gut ecosystem, are based on sequencing of the 16S ribosomal gene, which allows for identification of taxonomic composition of the microbiota. However, this approach does not allow for mechanistic understanding of underlying metabolic processes that could affect the host. Whole genome metagenomic sequencing has provided knowledge about the structure of the human gut microbiome and identified a large number of genes and direct links to functional information (9, 10). Going beyond traditional comparative analysis of functional components, by integrating metagenomic data with metabolic network analysis, provides deeper understanding of metabolic capabilities of the microbiome (11), and this approach could be very useful for mechanistically delineating the link between the gut microbiome and human health.

We studied metagenomes of 12 symptomatic atherosclerosis patients and 13 matched controls. The analyses were performed with a newly developed bioinformatics pipeline that allows for analysis of metagenomic sequence reads, *de novo* assembly and identification of enriched functional features in the context of a metabolic pathway.

Using this pipeline, to our surprise we identified 82 species in all 25 subjects that form the core microbiota in this cohort.

Furthermore, we found that the metagenome of the patients is enriched in genes associated with peptidoglycan biosynthesis, and this finding show that increased peptidoglycan production by the gut metagenome can contribute to symptomatic atherosclerosis. The increased abundance of genes in this pathway cannot be explained solely by a general increase in Gram-positive bacteria because both Gram-positive and Gram-negative bacteria have peptidoglycan and even more, abundant Gram- positive groups of bacteria such as *Eubacterium* and *Roseburia* were enriched in controls. Given the previous observation that peptidoglycan is important for priming the innate immune system and enhancing neutrophil function systemically (21), we show that increased inflammation in the host is the underlying mechanism linking enriched peptidoglycan genes to symptomatic atherosclerosis. Enriched levels of phytoene dehydrogenase in controls and its association with elevated levels of β-carotene in the serum which indicate that the production of this anti-oxidant by the gut microbiota has a positive health benefit for the host. And can be used to design a prevention strategy for people at risk. Lycopene and β-carotene are associated with a reduced risk of CVD in epidemiological studies (23, 24), but several large randomized, placebo-controlled studies with durations up to 12 years have failed to show that pure β-carotene treatment reduces CVD risk (25, 26). However, lycopene has been related to intima-media thickness of the common carotid artery (27) and suggested to play a role in the early stage and prevention of atherosclerosis (28). A previous study encompassing more than 500 subjects failed to observe an association between lycopene intake and plasma lycopene levels (29), indicating that other mechanisms might be more important or efficient than oral intake of lycopene.

Together with evidence that bacterial species from the human gut can synthesize carotenoids (30, 31), our findings of increased prevalence of phytoene dehydrogenase, and increased levels of β-carotene in plasma of control subjects represent important steps towards elucidating the importance of carotenoids in the development of atherosclerosis and is the basis for one object of the invention herein. It is worth noting that peptidoglycan and phytoene dehydrogenase genes were not linked to obesity since there was no significant difference in abundance of these genes between lean and overweight/obese subjects in our study or in the meta-analysis of an independent study (14).

We conclude that in this study we identified several compositional and functional alterations of the gut metagenome to be related to symptomatic atherosclerosis. At the taxonomical level, we observed associations between enterotypes, genera and species, and symptomatic atherosclerosis. In the microbiome, genes in the peptidoglycan pathway were enriched in patients, while genes involved in synthesis of antioxidants (β-carotene) were enriched in control subjects and anti-inflammatory molecules (butyrate) negatively correlated with hsCRP, indicating that the metagenome contribute to the development of symptomatic atherosclerosis by acting as a regulator of the host inflammatory pathways.

A primary object of the present invention is to treat or prevent atherosclerosis by giving β-carotene supplements or probiotic bacteria producing β-carotene to a patient having cardiovascular disease, or increased risk thereof, such as atherosclerosis and atherosclerotic associated disease based on the analysis of the presence of specific bacterial groups or species in the gut flora of a person to be used alone, or in combination with other measurements such as blood cholesterol and blood pressure. Such probiotic bacteria could be any probiotic strain producing β-carotene, especially a *Lactobacillus reuteri* strain. Such probiotic bacteria could be given in a suitable amount for example in the range of 10³ to 10¹² CFU per day, especially 10⁵ to 10⁹ CFU per day.

Another object of the present invention is to analyze the presence of specific bacterial groups or species in the gut flora of a person to be used alone, or in combination with other measurements such as blood cholesterol and blood pressure, to predict a person's risk of having cardiovascular disease such as atherosclerosis and atherosclerotic associated disease.

An object of the present invention is to treat a patient having cardiovascular disease, or increased risk thereof, such as atherosclerosis and atherosclerotic associated disease based on the analysis of the presence of bacteria of the genus *Collinsella* in the gut flora of a person, said treatment for example using a suitable antimicrobial or vaccine to reduce or eradicate such bacteria.

Another object of the present invention is to analyze the presence of bacteria of the genus *Collinsella* in the gut flora of a person to be used alone, or in combination with other measurements such as blood cholesterol and blood pressure, to predict a person's risk of having cardiovascular disease such as atherosclerosis and atherosclerotic associated disease.

An object of the present invention is to treat a patient having cardiovascular disease, or increased risk thereof, such as atherosclerosis and atherosclerotic associated disease based on the analysis of the presence of phytone dehydrogenase gene in the gut flora of such person.

Another object of the present invention is to analyze the presence of phytoene dehydrogenase genes in the gut flora of a person to be used alone, or in combination with other measurements such as blood cholesterol and blood pressure, to predict the person's risk of having cardiovascular disease such as atherosclerosis and atherosclerotic associated disease.

An object of the present invention is to treat a patient having cardiovascular disease, or increased risk thereof, such as atherosclerosis and atherosclerotic associated disease based on the analysis of serum levels of β-carotene. Such treatment or preventative strategy includes giving a person at risk β-carotene as such or in various matrixes or supplements or as giving probiotic bacteria, such as lactic acid bacteria, producing β-carotene.

Another object of the present invention is to analyze the serum levels of β-carotene to be used alone, or in combination with other measurements such as blood cholesterol and blood pressure, to predict the person's risk of having cardiovascular disease such as atherosclerosis and atherosclerotic associated disease.

Another object is to prevent from the risk of different cardiovascular disease, such as atherosclerosis, in a person having high bacterial amounts of specific groups or species in the gut microbiota, by using selected anti-microbial treatment.

Another object is to prevent from the risk of cardiovascular disease, such as atherosclerosis, in a person having high bacterial amounts of specific groups or species in the gut microbiota, by using vaccination to decrease said specific groups or species in the oral microbiota.

It is a further object of the invention to provide products for said identification, vaccination, eradication or immune intervention.

In embodiments of the invention where compositional alterations of the gut metagenome or microbiota are identified between disease subjects (patients) and control subjects, preferably this is carried out by identifying alterations in the type or amount of bacterial groups or species which are present, for example an alteration in species or other taxonomical abundance.

As such analysis is carried out using the gut metagenome (which allows the analysis of genetic material derived from complete microbial communities as opposed to single strains), the analysis of bacteria is carried out at a genome level, for example at the nucleic acid level using sequence based techniques, e.g. using alignments to reference bacterial genomes, e.g. as described in the experimental Examples. Such an analysis allows a correlation to be made between types or amounts of bacterial groups or species (and other taxonomical data) with either diseased subjects or control subjects and identifying differences between the two groups. For example, as shown herein, certain bacterial groups or species are enriched or depleted in the gut metagenome of disease subjects and others are enriched or depleted in the gut metagenome of control subjects. Generally such correlations will be identified using appropriate statistical analysis, e.g. as described in the Examples.

In embodiments of the invention where functional alterations of the gut metagenome are identified between disease subjects (patients) and control subjects, preferably this is carried out by identifying alterations in the type or amount of genes which are present in the metagenome, for example an alteration in gene (or ortholog) abundance. Again, as such analysis to provide functional information is carried out using the metagenome, the analysis is generally carried out at the nucleic acid level using sequence based techniques (for example whole genome metagenomic sequencing) e.g. using alignments to reference genes and/or by *de novo* assembly of genes, e.g. as described in the Examples, in order to potentially identify new genes in the metagenome that may be associated with disease. For example, shotgun sequencing would be a possible technique to use.

Preferably, said functional alteration is an alteration in a gene involved in metabolic function (metabolic function gene), in which case an analysis technique is selected which can analyse the levels of genes associated with particular functional metabolic pathways or metabolic functions, for example by mapping the metagenome onto a metabolic map, or otherwise integrating metagenomic data (e.g. in the form of relative gene abundance) with metabolic network analysis (e.g. using a database such as the KEGG database described in the Examples). For example, the results presented herein are based on an analysis of the abundance of various genes involved in the peptidoglycan synthesis pathway, the β-oxidation pathway, and other pathways, and being able to correlate the results with either diseased subjects or control subjects and identifying differences between the two groups. For example, certain genes are enriched (here for example genes encoding for or associated with peptidoglycan synthesis) or depleted (here for example genes encoding phytoene dehydrogenase) in the gut metagenome of patients and certain genes are enriched (here for example genes encoding phytoene dehydrogenase or other genes involved in the synthesis of anti-oxidants such as β-carotene) or depleted in the gut metagenomes of control subjects. Generally such correlations will be identified using appropriate statistical analysis, e.g. as described in the Examples.

In preferred embodiments both compositional (e.g. taxonomic) and functional alterations related to atherosclerosis or atherosclerotic associated disease will be identified.

In these embodiments, disease subjects (also referred to as patients) will generally be those exhibiting symptomatic atherosclerosis, e.g. patients with symptomatic and detectable atherosclerotic plaques, for example in the carotid artery. Other features of exemplary disease subjects for such metagenomic analysis are described in the Examples.

Preferably the level of the biomarker (e.g. gene) or bacteria in question is determined by analysing a test sample which is obtained or removed from said subject by an appropriate means. The methods and uses of the invention are thus generally carried out *in vitro* on biological samples obtained from an appropriate subject. The appropriate biological sample will depend on the analysis to be carried out. For example, if the analysis is of the gut metagenome or gut flora then an appropriate biological sample might be a fecal sample or an intestinal sample such as an intestinal biopsy sample, whereas in other methods e.g. methods and uses where the levels of β-carotene are analysed, then analysis is preferably carried out on a body fluid, more preferably a circulatory body fluid such as blood (including all blood derived components, for example plasma, serum etc.). An especially preferred body fluid is blood or a blood component, in particular serum.

As described elsewhere herein, the present invention provides various methods for determining if a subject is in a risk group for developing atherosclerosis or atherosclerotic associated disease, or if a subject has atherosclerosis or atherosclerotic associated disease. Put another way, such methods can be viewed as methods of predicting a subject's risk of having atherosclerosis or atherosclerotic associated disease, or methods of diagnosing whether a subject is in a risk group for developing atherosclerosis or atherosclerotic associated disease, or diagnosing if a subject has atherosclerosis or atherosclerotic associated disease.

Thus, exemplary subjects (also sometimes referred to herein as test subjects) to undergo such methods would be subjects that have or are suspected of having atherosclerosis or atherosclerotic associated disease, or subjects that are believed to be in a risk group or otherwise at risk for developing atherosclerosis or atherosclerotic associated disease.

Preferred methods comprise analysing a sample of gut flora from said subject for the presence of specific bacterial groups or species, for example analysing said sample for the enrichment or increase in certain bacterial groups or species compared to a control level, in particular those species which have been identified as being enriched or increased in the gut metagenome of subjects with atherosclerosis or atherosclerotic associated disease. Alternatively, for bacterial groups or species that have been identified as being enriched or increased in the gut metagenome of control subjects (healthy subjects), the sample, e.g. the gut flora sample, from the test subject might be analysed for the reduction or depletion in certain bacterial groups or species, compared to a control level.

In one embodiment of the methods of the invention the bacteria analysed is of the genus *Collinsella.* In such methods, an increased level of *Collinsella* bacteria compared to a control level is indicative of the subject having an increased risk of atherosclerosis or atherosclerotic associated disease or having atherosclerosis or atherosclerotic associated disease.

In other embodiments of the methods of the invention the bacteria analysed is of the genus *Roseburia* or *Eubacterium.* In such methods, a reduced or decreased level of *Roseburia* or *Eubacterium* bacteria compared to a control level is indicative of the subject having an increased risk of atherosclerosis or atherosclerotic associated disease or having atherosclerosis or atherosclerotic associated disease.

In preferred aspects the bacteria or gut flora of said subject is analysed for the presence of one or more phytoene dehydrogenase genes, and wherein a reduced level of said phytoene dehydrogenase genes compared to a control level is indicative of the subject having an increased risk of atherosclerosis or atherosclerotic associated disease or having atherosclerosis or atherosclerotic associated disease.

In other preferred aspects, the bacteria or gut flora of said subject is analysed for the presence of one or more peptidoglycan genes, and wherein an increased level of said peptidoglycan genes compared to a control level is indicative of the subject having an increased risk of atherosclerosis or atherosclerotic associated disease or having atherosclerosis or atherosclerotic associated disease.

In other preferred aspects, the invention provides a method for determining if a subject is in a risk group for developing atherosclerosis or atherosclerotic associated disease or if a subject has atherosclerosis or atherosclerotic associated disease, said method comprising analysing the gut flora of said subject for the presence of one or more phytoene dehydrogenase genes, preferably wherein a reduced level of said phytoene dehydrogenase genes compared to a control level is indicative of the subject having an increased risk of atherosclerosis or atherosclerotic associated disease or having atherosclerosis or atherosclerotic associated disease.

In other preferred aspects, the invention provides a method for determining if a subject is in a risk group for developing atherosclerosis or atherosclerotic associated disease or if a subject has atherosclerosis or atherosclerotic associated disease, said method comprising analysing the levels of β-carotene in the serum of said subject, preferably wherein a decreased level of β-carotene compared to a control level is indicative of the subject having an increased risk of atherosclerosis or atherosclerotic associated disease or having atherosclerosis or atherosclerotic associated disease.

In other preferred aspects, the invention provides a method for determining if a subject is in a risk group for developing atherosclerosis or atherosclerotic associated disease or if a subject has atherosclerosis or atherosclerotic associated disease, said method comprising analysing the gut flora of said subject for the presence of one or more peptidoglycan genes, preferably wherein an increased level of said peptidoglycan genes compared to a control level is indicative of the subject having an increased risk of atherosclerosis or atherosclerotic associated disease or having atherosclerosis or atherosclerotic associated disease.

Peptidoglycan genes as referred to herein include genes associated with peptidoglycan biosynthesis/genes in the peptidoglycan pathway, for example genes encoding proteins in the peptidoglycan biosynthesis pathway or genes encoding proteins involved in peptidoglycan synthesis, including precursors or metabolites, see for example the pathway and KOs shown in Figure 3A.

The methods of determining (methods of diagnosis) of the present invention thus allow the identification or diagnosis of a subject as having an increased risk of atherosclerosis or atherosclerotic associated disease, or having atherosclerosis or atherosclerotic associated disease. Such identification or diagnosis of a subject having an increased risk of atherosclerosis or atherosclerotic associated disease, or having atherosclerosis or atherosclerotic associated disease, can thus be a further step in the methods of the invention. Other further steps include assaying a sample for bacteria or genes using appropriate methods, for example as described herein (e.g. at the nucleic acid level using sequencing or a sequence based technique, for example after isolating nucleic acid from the sample, or any other appropriate assay as described herein). Other further steps include assaying a sample for β-carotene using appropriate methods, for example as described herein.

Thus, it can be seen that the diagnostic methods of the invention involve a determination or measurement of the level or amount of certain bacteria or genes. The methods may optionally comprise comparing the level or amount of the relevant bacteria or genes found in said subject (test subject) to a control level.

It should be noted however that although the control level for comparison would generally be derived by testing an appropriate set of control subjects, the methods of the invention would not necessarily involve carrying out active tests on such a set of control subjects but would generally involve a comparison with a control level which had been determined previously from control subjects.

The diagnostic methods of the invention can be used to identify subjects with symptomatic atherosclerosis or atherosclerotic associated disease or can be used for early identification of subjects which are at risk for developing atherosclerosis or atherosclerotic associated disease, for example subjects which are asymptomatic for atherosclerosis or atherosclerotic associated disease.

The diagnostic methods of the invention can also be used to identify subjects requiring more intensive monitoring or subjects which might benefit from early therapeutic intervention for atherosclerosis or atherosclerotic associated disease, e.g. by surgery, pharmaceutical therapy, or non-pharmaceutical therapy. Thus, where a positive diagnosis or indication is made (i.e. a diagnosis or indication of atherosclerosis), the diagnostic methods of the invention may further comprise a step in which the subject is treated for atherosclerosis or atherosclerotic associated disease, for example by surgery, pharmaceutical therapy, or non-pharmaceutical therapy. Appropriate therapies for atherosclerosis or atherosclerotic associated disease would be well known and described in the art. By way of example, non-pharmaceutical therapy would include changes to diet, cessation of smoking or regular exercise. Pharmaceutical therapy would include for example the administration of statins. Surgical therapy would include angioplasty, the use of stents or bypass surgery. Alternatively, or in addition, β-carotene therapy or treatment with an antimicrobial agent or vaccine as described herein could be used.

Thus, yet further aspects of the invention provide a method of treating atherosclerosis or atherosclerotic associated disease in a subject comprising: carrying out surgery or non-pharmaceutical therapy, or administering an effective amount of an appropriate pharmaceutical agent, to a subject having atherosclerosis or atherosclerotic associated disease, wherein said subject is determined to have or to be at risk of atherosclerosis or atherosclerotic associated disease using a diagnostic method of the present invention.

The diagnostic methods of the invention can also be used to monitor the progress of atherosclerosis or atherosclerotic associated disease in a subject. Such monitoring can take place before, during or after treatment of atherosclerosis or atherosclerotic associated disease by surgery or therapy.

For example, subsequent to such surgery or therapy, the diagnostic methods of the present invention can be used to monitor the progress of atherosclerosis or atherosclerotic associated disease, to assess the effectiveness of therapy or to monitor the progress of therapy, i.e. can be used for active monitoring of therapy. In such cases serial (periodic) measurement of levels of the relevant biomarker (bacteria or gene) and monitoring for a change in said biomarker levels will allow the assessment of whether or not, or the extent to which, surgery or therapy for atherosclerosis or atherosclerotic associated disease has been effective, or whether or not atherosclerosis or atherosclerotic associated disease is re-occurring or worsening in the subject.

Such monitoring can even be carried out on a healthy individual, for example an individual who is thought to be at risk of developing atherosclerosis or atherosclerotic associated disease, in order to obtain an early and ideally pre-clinical indication of atherosclerosis or atherosclerotic associated disease.

Generally, in such embodiments, an increase in the level of *Collinsella* bacteria or a peptidoglycan gene in the gut flora of a test subject, or a decrease in the level of *Roseburia* or *Eubacterium* bacteria or a phytoene dehydrogenase gene in the gut flora of a test subject, or a decrease in the level of circulating β-carotene, for example serum levels of β-carotene, is indicative of progression of atherosclerosis or atherosclerotic associated disease or early signs of development of atherosclerosis or atherosclerotic associated disease.

Thus, the observed associations of increased or decreased levels of bacteria or genes of the gut metagenome with the presence of atherosclerosis or atherosclerotic associated disease will not only allow diagnosis but will also allow active monitoring of patients and their treatment to take place. Thus, the methods of the invention can be used to guide atherosclerosis or atherosclerotic associated disease management and preferably optimize therapy.

Although the diagnostic methods of the invention and the measurement of the above discussed biomarkers (e.g. bacteria and genes) can be used in isolation, equally they can form part of a multimarker approach for diagnosis. Thus, the diagnostic methods of the present invention might not only be used in place of the measurement of other biomarkers (i.e. be used as single markers), but might also be used in combination, or in addition to the measurement of one or more other markers or biomarkers known to be associated with cardiovascular disease, for example atherosclerosis or atherosclerotic associated disease (i.e. in a multimarker assay).

Thus, preferred methods of the invention further comprise the measurement or determination of one or more other markers of atherosclerosis or atherosclerotic associated diseases, or of cardiovascular disease. Such other markers might be any of those already documented or known in the art and include blood cholesterol or blood pressure or waist circumference.

The "increase" in the levels or "increased" level or "enrichment", etc.,of bacteria or genes as described herein includes any measurable increase or elevation or enrichment of the marker in question when the marker in question is compared with a control level. Preferably the increase in level will be significant, more preferably clinically or statistically significant (preferably with a probability value of <0.05), most preferably clinically and statistically significant (preferably with a probability value of <0.05).

Similarly, the "decrease" in the levels or "decreasing" level, or "depletion", "reduction", etc., of bacteria, genes, or β-carotene, as described herein includes any measurable decrease or reduction or depletion of the marker in question when the marker in question is compared with a control level. Preferably, the decrease in level will be significant, more preferably clinically or statistically significant (preferably with a probability value of <0.05), most preferably clinically and statistically significant (preferably with a probability value of <0.05).

Methods of determining the statistical significance of differences in levels of a particular biomarker are well known and documented in the art. For example herein an increase or decrease in level of a particular biomarker is generally regarded as significant if a statistical comparison using an appropriate significance test shows a probability value of <0.05. More detail on appropriate methods of statistical analysis is provided in the Examples.

Said control level may correspond to the level of the equivalent biomarker in appropriate control subjects or samples. Alternatively, said control level may correspond to the level of the biomarker in question in the same individual subject, or a sample from said subject, measured at an earlier time point (e.g. comparison with a "baseline" level in that subject). This type of control level (i.e. a control level from an individual subject) is particularly useful for embodiments of the invention where serial or periodic measurements of the described biomarkers in individuals, either healthy or ill, are taken looking for changes in the levels of the biomarkers. In this regard, an appropriate control level will be the individual's own baseline, stable, or previous value (as appropriate) as opposed to a control level found in the general population.

Appropriate control subjects or samples for use in the methods of the invention would be readily identified by a person skilled in the art. Preferred control subjects would include healthy subjects (also referred to herein as healthy controls), for example, individuals who have no history of any form of cardiovascular disease, in particular atherosclerosis or atherosclerotic associated disease, and no other concurrent disease, or individuals with no current cardiovascular disease, in particular no atherosclerosis or atherosclerotic associated disease, for example no atherosclerotic plaques. Preferably control subjects are not regular users of any medication. Appropriate controls will generally also be age and/or sex matched. Other exemplary features of appropriate control subjects are described in the Examples, see e.g. Table 1.

Levels of bacteria or genes or β-carotene in a sample, e.g. in a gut flora sample, e.g. in a fecal sample or an intestinal sample such as an intestinal biopsy, or in a sample of body fluid, e.g. in a blood, serum or plasma sample, can be measured by any appropriate assay, a number of which are well known and documented in the art.

For example, conveniently, the levels of particular bacteria in a sample, for example in a gut flora sample such as a fecal sample or intestinal sample could be analysed at the nucleic acid level by sequence based techniques.

This invention can be practiced for example by using barcoded multiplexed-454 sequencing to analyze the bacterial composition of the gut microbiota, alone or in combination with other analysis such as blood cholesterol, blood pressure levels, and/or waist circumference, etc. in persons at risk for cardiovascular disease. The deep sequencing allows for a comprehensive description of microbial communities associated with cardiovascular disease such as atherosclerotic plaques.

The invention can also be practised using other methods for quantification of specific bacterial species or groups known in the art. These methods include, but are not limited to, quantitative PCR, ELISA, microarrays etc.

Similar techniques are appropriate for analysing the level of a particular gene in a sample (for example a phytoene dehydrogenase gene or a peptidoglycan gene).

The decrease or reduction of bacterial amounts of specific groups or species in the gut flora to beneficial levels or the eradication of bacteria may be accomplished by several suitable means generally known in the art. In one embodiment, an antibiotic having efficacy against these bacteria in the flora may be administered. The susceptibility of the targeted species to the selected antibiotics can be determined based on culture methods or genome screening.

The actual effective amounts of compounds (e.g. antimicrobial agents or vaccines) comprising a specific reduction of bacteria of the gut microbiota of the invention or the actual effective amount of β-carotene (e.g. in the form of β-carotene *per se* or a β-carotene supplement or probiotic bacteria) can and will vary according to the specific compounds being utilized, the mode of administration, and the age, weight and condition of the subject. Dosages for a particular individual subject can be determined by one of ordinary skill in the art using conventional considerations.

The present invention also encompasses use of the microbiome as a biomarker to construct microbiome profiles. Generally speaking, a microbiome profile is comprised of a plurality of values with each value representing the abundance of a microbiome biomolecule. The abundance of a microbiome biomolecule may be determined, for instance, by sequencing the nucleic acids of the microbiome as detailed in the examples. This sequencing data may then be analyzed by known software, as shown below.

A profile may be digitally-encoded on a computer-readable medium. The term "computer-readable medium" as used herein refers to any medium that participates in providing instructions to a processor for execution. Such a medium may take many forms, including but not limited to non-volatile media, volatile media, and transmission media. Non-volatile media may include, for example, optical or magnetic disks. Volatile media may include dynamic memory. Transmission media may include coaxial cables, copper wire and fiber optics. Transmission media may also take the form of acoustic, optical, or electromagnetic waves, such as those generated during radio frequency (RF) and infrared (IR) data communications. Common forms of computer-readable media include, for example, a diskette, hard disk, magnetic tape, or other magnetic medium, a CD-ROM, CDRW, DVD, or other optical medium, a RAM, a PROM, and EPROM, a FLASH-EPROM, or other memory chip or cartridge, a carrier wave, or other medium from which a computer can read. A particular profile may be coupled with additional data about that profile on a computer readable medium. For instance, a profile may be coupled with data to analyze if the person is within a risk group, or for intervention; what therapeutics, compounds, or drugs may be efficacious for that profile. Conversely, a profile may be coupled with data about what therapeutics, compounds, or drugs may not be efficacious for that profile.

The microbiome profile from the host may be determined using DNA sequencing according to the invention. The reference profiles may be stored on a computer-readable medium such that software known in the art and detailed in the examples may be used to compare the microbiome profile and the reference profiles.

The term "metagenomics" refers to the application of modern genomics techniques to the study of communities of microbial organisms directly in their natural environments, bypassing the need for isolation and lab cultivation of individual species.

As described elsewhere herein, the present invention provides methods of treating or preventing atherosclerosis or atherosclerotic associated disease in a subject having or at risk of having atherosclerosis or atherosclerotic associated disease, comprising the administration of an effective amount of β-carotene to said subject.

Thus, another preferred aspect of the invention provides β-carotene for use in the treatment or prevention of atherosclerosis or atherosclerotic associated disease in a subject having or at risk of having atherosclerosis or atherosclerotic associated disease.

Another preferred aspect is the use of β-carotene in the manufacture of a composition or medicament for use in the treatment or prevention of atherosclerosis or atherosclerotic associated disease in a subject having or at risk of having atherosclerosis or atherosclerotic associated disease.

In these embodiments, β-carotene may be administered as the molecule *per se*, or may be administered in the form of a β-carotene containing supplement or in the form of a probiotic bacteria producing β-carotene. Preferably, said supplement or probiotic bacteria comprises a *Lactobacillus reuteri* strain producing β-carotene.

These embodiments may comprise a further step in which a diagnostic method of the invention is carried out. Thus, such embodiments may involve a step in which the gut flora of said patient is analysed for the presence of one or more phytoene dehydrogenase genes, preferably wherein a reduced level of said phytoene dehydrogenase genes is indicative of the subject being able to benefit from β-carotene administration, and optionally then administering β-carotene.

Alternatively, such embodiments may involve a step in which a blood sample, preferably serum, of said patient is analysed for the presence of β-carotene, preferably wherein a decreased level of said β-carotene is indicative of the subject being able to benefit from β-carotene administration, and optionally then administering β-carotene.

As described elsewhere herein, the present invention also provides methods of treating or preventing atherosclerosis or atherosclerotic associated disease in a subject having or at risk of having atherosclerosis or atherosclerotic associated disease, comprising the administration of an effective amount of an antimicrobial agent or vaccine to said subject.

Thus, another preferred aspect of the invention provides an antimicrobial agent or vaccine for use in the treatment or prevention of atherosclerosis or atherosclerotic associated disease in a subject having or at risk of having atherosclerosis or atherosclerotic associated disease, wherein said agent or vaccine has the effect of reducing levels of bacteria which are elevated in the gut flora of the subject compared to the level in a control subject.

Another preferred aspect is the use of an antimicrobial agent or vaccine in the manufacture of a composition or medicament for use in the treatment or prevention of atherosclerosis or atherosclerotic associated disease in a subject having or at risk of having atherosclerosis or atherosclerotic associated disease, wherein said agent or vaccine has the effect of reducing levels of bacteria which are elevated in the gut flora of the subject compared to the level in a control subject.

These embodiments may comprise a further step in which a diagnostic method of the invention is carried out. Thus, such embodiments may involve a step of detecting the presence of bacteria in the gut flora of said subject, and, where levels of said bacteria are elevated, this is indicative of the subject being able to benefit from administration of antimicrobial agent or vaccine, and optionally then administering an effective amount of an appropriate antimicrobial agent or vaccine. In preferred embodiments the bacteria analysed are *Collinsella* bacteria. Thus, preferred agents or vaccines will be those which can reduce or eradicate *Collinsella* bacteria.

As described elsewhere herein, appropriate antimicrobial agents or vaccines to be used in such embodiments will be readily identified by the skilled person, for example from the antimicrobial agents or vaccines available in the art, depending on the bacteria which is desired to be reduced or eradicated. The appropriateness of agents can readily be tested for their ability to inhibit bacterial growth, for example using appropriate *in vitro* assays.

The therapeutic uses of the invention as defined herein include the reduction, prevention or alleviation of the relevant disorder or symptoms of disorder (e.g. can result in the modulation of disease symptoms). Such reduction, prevention or alleviation of a disorder or symptoms thereof can be measured by any appropriate assay. Preferably the reduction or alleviation of a disorder or symptoms is clinically and/or statistically significant, preferably with a probability value of <0.05. Such reduction or alleviation of a disorder or symptoms are generally determined compared to an appropriate control subject or population, for example a healthy subject or an untreated or placebo treated subject.

An appropriate mode of administration and formulation of the therapeutic agent is chosen depending on the treatment. A preferred mode of administration for probiotic bacteria or other supplements is oral or rectal, however, equally for some treatments intravenous or intramuscular injection will be appropriate.

Appropriate doses of the therapeutic agents as defined herein can be chosen by standard methods depending on the particular agent, the age, weight and condition of the patient, the mode of administration and the formulation concerned.

The therapeutic and diagnostic methods of the invention as described herein can be carried out on any type of subject which is capable of suffering from atherosclerosis or atherosclerotic associated disease. The methods are generally carried out on mammals, preferably humans.

The following are some examples of the invention, which are not meant to be limiting of the use of the invention herein but to show practical examples in detail how the invention may be used.

### EXAMPLE 1

### Patient and control groups

The patient samples were from the Goteborg Atheroma Study Group Biobank, which includes samples from patients who had undergone surgery to excise an atherosclerotic plaque (12). All patients had severely stenotic plaques in the carotid artery with ipsilateral manifestations of emboli to either the brain, as minor brain infarction or transient ischemic symptoms, or to the retinal artery (Table 1). The control group was selected to represent an age- and sex-matched group with no cardiovascular health problems and was recruited from two on-going population-based cohorts that have been described previously (32, 33). The investigations of the control group included repeated ultrasound examinations of the carotid and femoral arteries, and no large, potentially vulnerable plaques were detected. Further inclusion criteria in the control group were no history of cardiovascular disease, no smoking, no diabetes and no treated hyperlipidemia. The underlying rationale was to avoid subjects with vulnerable plaques defined as echothin plaques with stenosis above 50% of vessel lumen (34, 35). Analysis of updated health records showed that one control subject had a dilation of ascending aorta since the initial recruitment as "healthy control" and a second had white matter disease in the brain, possibly due to a small artery disease. As these diagnoses may have atherosclerosis as underlying cause, we excluded these subjects from analyses of differences between patients and controls, although they were included in specified analyses of the total cohort.
Blood samples were drawn before surgery and plasma and serum samples were prepared and immediately frozen at -70 °C. The subjects were given material and instructions for providing fecal samples at home. Methods for processing fecal samples and isolation of metagenomic DNA have been described previously (36).

**Table 1. Characteristics of study subjects.**

| | Controls (n = 13) | Patients (n = 12) | P value C vs. P | Excluded controls (n = 2) |
|---|---|---|---|---|
| Males, n | 10 | 9 | | 2 |
| Age, years | 70.5 (0.5) | 67.6 (8.6) | 0.27^{#} | 71(0) |
| Current smoker, n | 0 | 4 | | 0 |
| Hypertension, n | 2 | 10 | | 0 |
| Diabetes, n | 0 | 3 | | 0 |
| Previous myocardial infarction, n | 0 | 3 | | 0 |
| Statin treatment, n | 0 | 9 | | 0 |
| Aspirin | 0 | 12 | | 0 |
| Cerebrovascular event | | | | |
| Minor brain infarction, n | 0 | 5 | | 0 |
| Transient ischemic symptoms ,n | 0 | 4 | | 0 |
| Retinal artery,n | 0 | 3 | | 0 |
| BMI, kg/m² | 23.7 (2.9)† | 25.8 (2.4)‡ | 0.08^{#} | 25.6 (8.8) |
| Cholesterol, mmol/L | 5.59 (1.20) | 4.62 (1.59) | 0.10^{#} | 5.38 (0.62) |
| Triglycerides, mmol/L | 1.19 (0.74) | 1.72 (1.08) | 0.04* | 1.8 (0.77) |
| HDL cholesterol, mmol/L | 1.67 (0.44) | 1.32 (0.26) | 0.026^{#} | 1.49 (0.63) |
| LDL cholesterol, mmol/L | 3.39 (1.05) | 2.53 (1.44) | 0.10^{#} | 3.07 (0.91) |
| ApoA1 | 1.44 (0.20) | 1.33 (0.21) | 0.22^{#} | 1.46 (1.48) |
| ApoB | 1.09 (0.29) | 0.95 (0.34) | 0.27^{#} | 1.16 (0.42) |
| WBC | 5.47 (1.06)‡ | 7.78 (1.56)† | <0.001 ^{#} | 5.05 (1.77) |
| hsCRP, mg/L | 2.14 (3.35) | 4.81 (5.95) | 0.12* | 1.81 (2.27) |

| | | | | |
|---|---|---|---|---|
| Data are mean (standard deviation) unless otherwise indicated. # Welch's t-test; * Wilcoxon rank sum test. †n=11, ‡n=10. | | | | |

We sequenced the gut metagenomes of 12 patients with symptomatic atherosclerotic plaques (who had undergone carotid endarterectomy for minor ischemic stroke, transient ischemic attack, or amaurosis fugax) and 13 gender- and age-matched controls without large vulnerable plaques in the carotid arteries. To analyze the data we developed and used a bioinformatics pipeline, MEDUSA (MEtagenomic Data UtiliSation and Analysis), that besides identification of species abundance also allows for de novo assembly and the identification of enriched metabolic functions in the metagenome. Using MEDUSA we demonstrated that it is possible to identify unique metabolic functions associated with the patients and controls, and that the functions identified based on metagenomics are associated with altered serum metabolites. Our study hereby represents a proof of principle that metagenomics can be used for identification of specific metabolic functions of the gut microbiota that is associated with previously known and unknown metabolites and markers in serum that may influence atherosclerotic disease prevention, onset and development.

The clinical definition of minor brain infarction corresponds to a patient who has only mild and not severe functional deficits, without any need of prolonged hospital care. Hence, the underlying etiology in all these patients was a vulnerable atherosclerotic plaque with plaque rupture and embolism leading to operations with excision of the plaque (12) and it is not likely that the clinical events per se would directly influence the gut metagenome (minor stroke has no acute effects on CRP and white blood cell count (13) and the patients only had transient or minor tissue-damaging effects in the brain or eye).

### EXAMPLE 2

### Sequencing.

All samples were sequenced in the Illumina HiSeq2000 instrument at SciLifeLab in Stockholm, Sweden, with up to 10 samples pooled in one lane. Libraries were prepared with a fragment length of approximately 300 bp. Paired-end reads were generated with 100 bp in the forward and reverse direction.

### Data quality control.

Sequencing adapter sequences were removed with Cutadapt M. Martin. Cutadapt removes adapter sequences from high-throughput sequencing reads. EMBnet.journal, North America, 17, May 2011. The length of each read was trimmed with SolexaQA (Cox, M.P., D.A. Peterson, and P.J. Biggs. 2010. SolexaQA: At-a-glance quality assessment of Illumina second-generation sequencing data. BMC Bioinformatics 11:485) with the options -b p-0.05 (37). Read pairs with either reads shorter than 35 bp were removed with a custom Python script. The high-quality reads aligning to the human genome (NCBI version 37) with Bowtie (38) using -n 2 -135 -e 200 --best -p 8 --chunkmbs 1024 -X 600 -tryhard were removed. The remaining set of high-quality non-human reads were then used for further analysis.

### Alignment to reference genomes and taxonomical analysis.

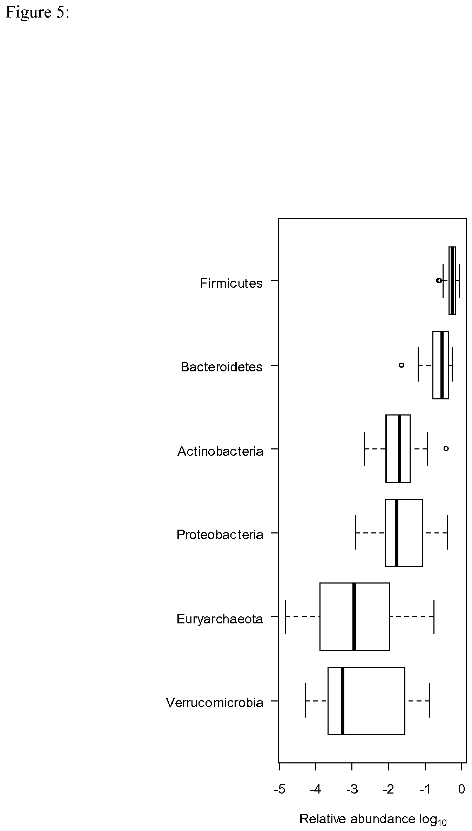

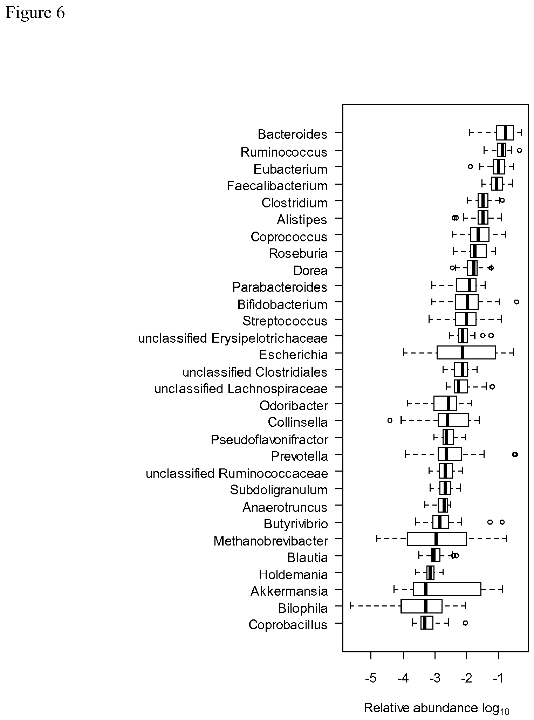

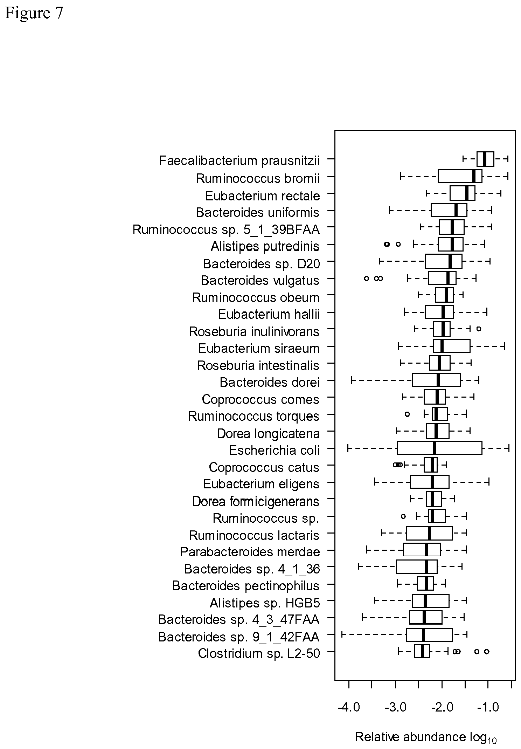

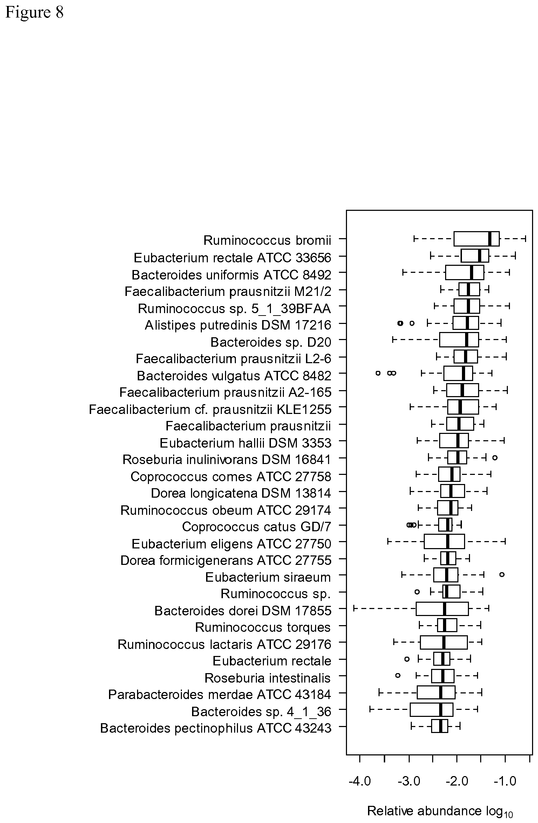

A set of 2382 of microbial reference genomes were obtained from National Center for Biological Information (NCBI) and Human Microbiome Project (HMP) on 2011-08-02. The reference genomes were combined into two Bowtie indexes and the metagenomic sequence reads were aligned to the reference genomes using Bowtie with parameters -n 2 -135 -e 200 --best -p 8 --chunkmbs 1024 -X 600 - tryhard. Mapping results were merged by selecting the alignment with fewest mismatches; if a read was aligned to a reference genome with the same number of mismatches, each genome was assigned 1/2 to each genome. The relative abundance of each genome was calculated by summing the number of reads aligned to that genome divided by the genome size. In each subject, the relative abundance was scaled to sum to one. The taxonomic rank for every genome was downloaded from NCBI taxonomy to assign each genome to a species, genus and phyla. The relative abundance for each taxonomical rank was calculated buy summing the relative abundance of all its members (Fig. 5,6,7,8).

### De novo assembly and gene calling.

The high-quality reads were used for *de novo* assembly with Velvet (39) into contigs of at least 500 bp length using 3 as coverage cutoff and kmer length of 31. To obtain long contigs with high specificity, we iteratively explored parameter values for the kmer length and coverage cutoff to balance the total assembly length and the N50 value to be used in the final *de novo* assembly. Reads from each subject were used in separate assemblies and unassembled reads were then used in a global final assembly. Genes were predicted on the contigs with MetaGeneMark (17). All genes were then aligned on the contigset with Bowtie using the same parameters as above. The abundance of a gene was calculated by counting the number of reads that align to the gene normalizing by the gene length and the total number of reads aligned to any contig.

### Gene annotation.

The genes were annotated to the KEGG database with hidden Markov models (HMMs). Protein sequences for microbioal orthologs were downloaded and aligned with MUSCLE (40). HMMs were generated with HMMer3 (41) for each KO. Each gene was queried on the 4283 HMMs and annotated the KO with lowest scoring E-value below 10-20. Out of the 2,645,414 genes, 848,353 (32%) were annotated to KOs. The genes were also annotated to carbohydrate active enzymes (CAZy) (42). The CAZy proteins of bacterial and archaeal origin were downloaded and HMMs were built and genes annotated as described above. The feature abundance (KOs and CAZy) was calculated by summing the abundance of genes annotated to a feature.

Genes for betaine reductase were collected from two species: Gi: 126699967, 126699969 from *Clostridium difficile* 630 and Gi: 78044558, 78044225 *Carboxydothermus hydrogenoformans* Z-2901. The gene catalogue was searched against these four genes with USEARCH 10 using an E-value cutoff of 10-30.

### Statistical analysis.

To determine differential abundance of metagenomic features (i.e. taxonomic and functional features between patients and controls) Wilcoxon rank sum test was applied. Strains and genera with a relative abundance in any subject above 10-5 and 10-3, respectively, were included in the analysis. Correlations were done between serum biomarkers and metagenomic features with spearman's correlation. P values were adjusted with False Discovery Rate (FDR) with the method from Benjamini and Hockberg (43) when multiple hypotheses were considered simultaneously and are denoted Adj. P. The R package ade4 using instrumental principal component analysis (44) was used to determine the global analysis of species abundance between patients and controls. Monte-Carlo test on the between-groups inertia percentage was performed 10,000 permutations to calculate a p value in Figure 1b.

### Measurement of β-carotene and lycopene.

β-carotene and lycopene were measured in the serum from healthy controls and patients using a modified protocol from Sowell et al. (Clin Chem 40, 411-416 (1994)). Briefly, 200 µl of serum was mixed with 200 µl of ethanol and 8 µl of 0.191 mmol/l retinyl-propionate in ethanol. Samples were vortexed gently and then 1 ml hexane was added; the samples were again vortexed (for 30 s). The phases were separated by centrifugation at 1500g for 5 min and 900 µl of the upper phase was then transferred to a new tube. The samples were dried under low pressure at room temperature in a Speedvac concentrator, not to complete dryness. The residue was dissolved in 100 µl ethanol followed by addition of 100 µl acetonitrile. Samples were protected from light during handling and preparation.

The compounds were measured using a Dionex HPLC system with a C18 column, kept at 29°C. The mobile phase was ethanol and acetonitrile (1:1) with 0.1 ml/l diethylamine and was kept at a flow rate of 0.9 ml/min. Samples were stored at 4°C before injection of 50 µl. Chromatograms for absorbance at the wavelengths 300, 325 and 450 nm were collected simultaneously for 20 min. Peaks were identified by comparing retention time with a standard solution of β-carotene and lycopene. Quantification was based on the area under the curve.

### Taxonomic characterization of the gut microbiota.

In total, we generated 337 million 100 bp paired-end reads (12.5±4.7 (SD) million reads per sample) that were first trimmed and filtered to only contain high quality non-human reads longer than 35 bp (Fig. 1a). To determine the composition of the gut microbiota, we aligned the reads to a catalog of 2382 non-redundant reference genomes collected from NCBI and HMP catalog (hmpdacc.org). On average, 28% of the reads in a sample could be aligned to any reference genome, which is close to the 31% found in a previous metagenomic study using Illumina reads (9). The majority (98±4% (SD) of aligned reads were bacterial and dominated by the phyla Firmicutes and Bacteroidetes, representing 56% and 29% of the microbiota, respectively, followed by Actinobacteria (6%) and Proteobacteria (4%). This distribution is in agreement with previous observations (14, 15). The archael phylum Euryarchaeota was also present but with a high inter-subject variation (2.0±4.3% (SD); and was dominated by the species *Methanobrevibacter smithii,* which constituted at least 93% of the reads assigned to Euryarchaeota in any individual. *Bacteroides, Ruminococcus, Eubacterium* and *Faecalibacterium* were the most abundant genera in our cohort as found previously (9, 14). Species and genome level abundances were also calculated and *Faecalibacterium prausnitzii* was shown to be the most abundant species. At coverage of at least 1% of aligned reads to reference genomes, we identified 82 species in all 27 subjects constituting the core microbiota in our cohort.

### EXAMPLE 3

### PCA and enterotypes in the cohort.

An instrumental principal component analysis with the health status as the instrumental variable revealed that the microbial species abundance separated patients and healthy controls (Fig. 1b, P=1e-4). The genus *Collinsella* was enriched in patients whereas *Eubacterium* and *Roseburia* and three species of *Bacteroides* were enriched in control subjects (Adj. P<0.05, Wilcoxon rank sum test) (Fig. 1c). Several bacterial groups correlated with cardiovascular risk factors (Fig. 1d); in particular in the genus *Clostridiales: Clostridum* sp. SS2/1 and the poorly characterized butyrate-producing *Clostridium* SSC/2 negatively correlated with the inflammatory marker high sensitivity C-reactive protein (hsCRP).

A recent study has suggested that the human gut microbiota can be stratified into three enterotypes of distinct microbial compositions (14). We analyzed our samples according to that study (14), calculated the Jensen-Shannon distance of the genera abundance, and clustered samples with partitioning around mediods. The Calinski-Harabasz index indicated that the optimal number of clusters was three. However, when the average silhouette index was used to assess the quality of the clusters, we saw the highest silhouette index with two clusters which has also been observed previously (16). We chose, however, to use three clusters as proposed in the publication by
Arumugam *et al.* (14), which is the largest enterotypes study to date. The three enterotypes that we observed were characterized by the same contributors at the genus level as shown previously: *Bacteroides* contributed to enterotype 1, *Prevotella* contributed to entrotype 2 and *Ruminococcus* contributed to enterotype 3 (Fig.2). As described previously (14), different additional contributors for the third enterotype were also found, and this is in agreement with the observation that this enterotype is rather being characterized by low levels of *Bacteroides* and *Prevotella.* To test whether the enterotypes were associated with disease status, we used Fisher's exact test and showed that patients were underrepresented in enterotype 1 (P=0.0048) and overrepresented in enterotype 3 (P=0.047).

### EXAMPLE 4

### Metabolic functions of the gut microbiota.

To discover new genes in the metagenomes, we used MEDUSA (Fig. 1a) to perform *de novo* assembly of the sequence data, first for each individual sample separately and subsequently for a pool of all the non-assembled data from the individual samples, to create one global gene catalog of our cohort. A total of 1.7 Gbp of contigs longer than 500 bp could be assembled and with a N50 value of 1.8 kbp using 3 as coverage cutoff and kmer of 31. MetaGeneMark (17) was used to predict genes from the contig set and 2.6 million ORFs representing 1.4 million non-redundant genes were found. The genes were functionally annotated to KEGG, Pfam and CAZy databases and their relative abundances were assessed. On average, 60% of the reads could be aligned to the set of contigs, which is substantially more than the percentage of reads (28%) that could be aligned to the reference genomes. This indicates that our gene catalog contains a majority of the sequenced microbiome.

A global analysis of the abundance of KEGG orthologies (KO) resulted in separation of the patient group from the control group . In total, 225 KOs were differentially abundant (Adj. P<0.05) using Wilcoxon rank sum test, illustrating that there were functional aspects of the gut metagenome associated with symptomatic atherosclerosis. Enriched metabolic functions in the metagenomes of patients and controls can be assessed by integrating the relative gene abundance with metabolic networks. We used the reporter feature algorithm (18, 19), and based on the KEGG metabolic network and the pathway associations for the KOs together with the adjusted P values, we identified reporter pathways that contained several significantly differentially abundant KOs. This method was additionally used to identify reporter metabolites which are defined as metabolites around which the enzymatic reactions with associated KO are differentially abundant. From this analysis we found peptidoglycan biosynthesis pathway to be the highest scoring reporter pathway: a total of eight peptidoglycan biosynthetic KOs were enriched in the gut metagenomes of patients and one gene was enriched in controls (Adj. P<0.05, Fig. 3A). Consequently, we also found several of the metabolites in the peptidoglycan pathway to be reporter metabolites, e.g. UDP-N-acetyl-D-glucosamine, which is a key precursor for peptidoglycan, indicating significant changes in KOs linked to these metabolites.

There were features of the metagenome that correlated negatively with inflammation, the highest scoring association being butyrate-acetoacetate CoA-transferase (K01036) with hsCRP (Spearman rho=-0.73, Adj. P=0.04). These findings are in agreement with a previous study that found butyrate in the gut to be an important negative regulator of systemic inflammation (20). To investigate the origin of the butyrate-acetoacetate CoA-transferase genes, we performed a BLASTP search and identified the source as *Clostridium* sp. SS2/1; as discussed above, this species also negatively correlated with the inflammation marker hsCRP.

A recent metabolomics study showed that three microbially modulated metabolites of dietary phosphatidylcholine metabolism (choline, trimethylamine N-oxide and betaine) are associated with cardiovascular disease (CVD) in humans (8), so we reconstructed the metabolic pathway from phosphatidylcholine to trimethylamine but did not observe any significant association of gene abundance in this pathway with atherosclerosis. However, we observed a positive correlation between plasma triglycerides and the abundance of several KOs in the pathway for fatty acid metabolism, specifically β-oxidation which suggests strong interactions between the gut microbiota and dietary components. We also observed that the GS-GOGAT system, which the microbiota uses for assimilation of nitrogen into amino acids, was significantly enriched in the patient group (Fig. 3c). In particular, the ATP-dependent reaction carried out by glutamine synthase (Adj. P= 0.035) and the glutamate synthase large and small subunits (Adj. P=0.013 and Adj. P=0.0074, respectively) were enriched in patient microbiota. The ATP-independent glutamate dehydrogenase was not found to be different between the groups.

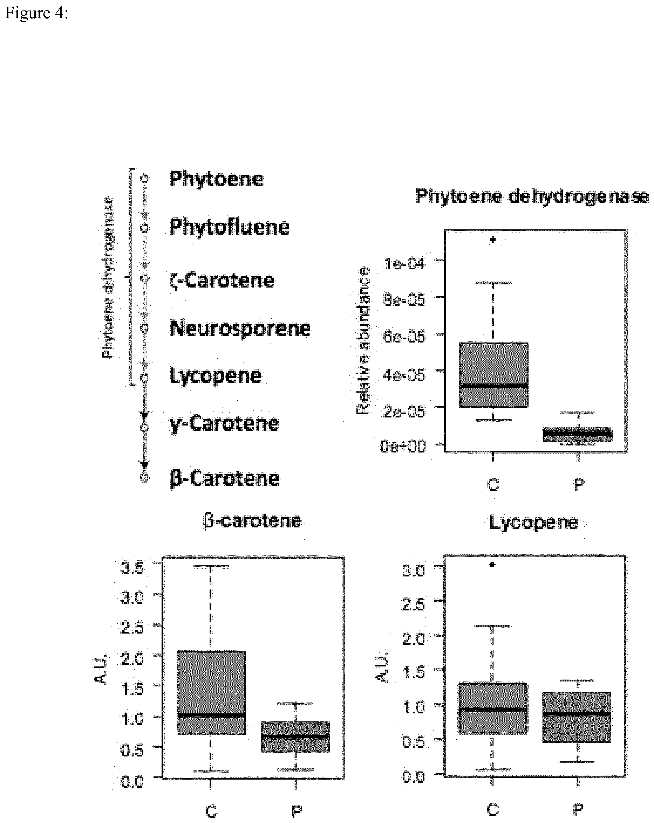

Interestingly, we also found phytoene dehydrogenase (K10027) to be the KO most significantly enriched in controls in our study (Adj. P=0.0046, Fig 4) which is a multi-functional enzyme involved in the metabolism of lipid-soluble antioxidants (such as the carotenoids lycopene and β-carotene). To determine the phylogenetic origin of the 13 genes annotated as phytoene dehydrogenases in this study, we used BLASTP to search for related sequences in the NCBI nr database. Seven of the genes matched to *Bacteroides,* two to *Clostridia,* two to *Prevotella* and the remaining two to Actinobacteria and various Bacteroidetes. The strong enrichment of phytoene dehydrogenase in the control group led us to speculate whether this may be associated with differences in lycopene or other carotenoids derived from this biosynthetic pathway, catalyzed by this enzyme. We therefore used HPLC analysis of serum samples to evaluate whether the enrichment of phytoene dehydrogenase was accompanied by increased levels of carotenoids, and indeed we found increased levels of β-carotene (P=0.05), but not lycopene, in serum of healthy controls compared with patients (Fig. 4).

### EXAMPLE 5

The method of the invention is used in a clinical setting to aid in the assessment if a person is in a risk group for developing cardiovascular disease, including arthrosclerosis and associated conditions. Faecal samples and blood samples are collected and other normal assessments such as blood pressure, BMI, waist size are made. The faecal and blood samples are processed according to the invention herein and a value is determined for the presence of genus *Collinsella* and / or phytoene dehydrogenase in the faecal sample and / or serum levels of β-carotene.

If bacteria of genus *Collinsella* are found, this alone or in combination with clinically used important values for the other variables, and also blood pressure, blood cholesterol etc, the person is considered at risk for having or developing a cardiovascular disease, including arthrosclerosis and should be treated according to clinical practise and also further monitored.

Embodiments of the invention are also described in the claims of the International application as filed:
1. The use of metagenomics to identify a compositional or functional alteration of the gut metagenome related to atherosclerosis or atherosclerotic associated disease.
2. The use of claim 1, wherein the metagenomics is whole genome metagenomics.
3. The use of claim 1 or claim 2, wherein said use comprises comparing the gut metagenome of subjects with atherosclerosis or atherosclerotic associated disease to the gut metagenome of control subjects and identifying differences between the metagenome of the disease subjects and the control subjects in the type or amount of microorganisms or the type or amount of genes.
4. The use of any one of claims 1 to 3, wherein the compositional alteration is an alteration in the type or amount of bacterial groups or species.
5. The use of any one of claims 1 to 3, wherein the functional alteration is an alteration in a metabolic function gene.
6. A method for determining if a subject is in a risk group for developing atherosclerosis or atherosclerotic associated disease, or if a subject has atherosclerosis or atherosclerotic associated disease, said method comprising analysing the gut flora of said subject for the presence of specific bacterial groups or species.
7. The method of claim 6, wherein the bacteria is of the genus *Collinsella.*
8. The method of claim 7, wherein an increased level of *Collinsella* bacteria compared to a control level is indicative of the subject having an increased risk of atherosclerosis or atherosclerotic associated disease or having atherosclerosis or atherosclerotic associated disease.
9. The method of claim 6, wherein the bacteria is of the genus *Roseburia* or *Eubacterium.*
10. The method of claim 9, wherein a reduced level of *Roseburia* or *Eubacterium* bacteria compared to a control level is indicative of the subject having an increased risk of atherosclerosis or atherosclerotic associated disease or having atherosclerosis or atherosclerotic associated disease.
11. The method of any one of claims 6, 9 or 10, wherein the bacteria are analysed for the presence of one or more phytoene dehydrogenase genes, and wherein a reduced level of said phytoene dehydrogenase genes compared to a control level is indicative of the subject having an increased risk of atherosclerosis or atherosclerotic associated disease or having atherosclerosis or atherosclerotic associated disease.
12. The method of any one of claims 6 to 8, wherein the bacteria are analysed for the presence of one or more peptidoglycan genes, and wherein an increased level of said peptidoglycan genes compared to a control level is indicative of the subject having an increased risk of atherosclerosis or atherosclerotic associated disease or having atherosclerosis or atherosclerotic associated disease.
13. A method for determining if a subject is in a risk group for developing atherosclerosis or atherosclerotic associated disease or if a subject has atherosclerosis or atherosclerotic associated disease, said method comprising analysing the gut flora of said subject for the presence of one or more phytoene dehydrogenase genes.
14. The method of claim 13, wherein a reduced level of said phytoene dehydrogenase genes compared to a control level is indicative of the subject having an increased risk of atherosclerosis or atherosclerotic associated disease or having atherosclerosis or atherosclerotic associated disease.
15. A method for determining if a subject is in a risk group for developing atherosclerosis or atherosclerotic associated disease or if a subject has atherosclerosis or atherosclerotic associated disease, said method comprising analysing the levels of β-carotene in the serum of said subject.
16. The method of claim 15, wherein a decreased level of β-carotene compared to a control level is indicative of the subject having an increased risk of atherosclerosis or atherosclerotic associated disease or having atherosclerosis or atherosclerotic associated disease.
17. A method for determining if a subject is in a risk group for developing atherosclerosis or atherosclerotic associated disease or if a subject has atherosclerosis or atherosclerotic associated disease, said method comprising analysing the gut flora of said subject for the presence of one or more peptidoglycan genes.
18. The method of claim 17, wherein an increased level of said peptidoglycan genes compared to a control level is indicative of the subject having an increased risk of atherosclerosis or atherosclerotic associated disease or having atherosclerosis or atherosclerotic associated disease.
19. The method of any one of claims 6 to 18, wherein one or more other markers of atherosclerosis or atherosclerotic associated disease are measured, for example blood cholesterol or blood pressure or waist circumference.
20. A method of treating or preventing atherosclerosis or atherosclerotic associated disease in a subject having or at risk of having atherosclerosis or atherosclerotic associated disease, comprising the administration of an effective amount of β-carotene.
21. The method of claim 20, comprising the administration of a β-carotene supplement or a probiotic bacteria producing β-carotene.
22. The method of claim 20 or claim 21, wherein said supplement or probiotic bacteria comprises a *Lactobacillus reuteri* strain producing β-carotene.
23. A method of treating or preventing atherosclerosis or atherosclerotic associated disease in a subject having or at risk of having atherosclerosis or atherosclerotic associated disease, comprising the administration of an effective amount of an antimicrobial agent or vaccine, wherein said agent or vaccine has the effect of reducing levels of bacteria which are elevated in the gut flora of the subject compared to the level in a control subject.
24. The method of claim 23, further comprising the step of detecting the presence of bacteria in the gut flora of said subject, and, where levels of said bacteria are elevated compared to the level in a control subject, administering an effective amount of an appropriate antimicrobial agent or vaccine.
25. The method of claim 23 or claim 24, wherein said bacteria are *Collinsella* bacteria.
26. β-carotene for use in treating or preventing atherosclerosis or atherosclerotic associated disease in a subject having or at risk of having atherosclerosis or atherosclerotic associated disease.
27. The β-carotene for use as claimed in claim 26, wherein said β-carotene is a β-carotene supplement or a probiotic bacteria producing β-carotene, preferably wherein said supplement or probiotic bacteria comprises a *Lactobacillus reuteri* strain producing β-carotene.
28. An antimicrobial agent or vaccine for use in treating or preventing atherosclerosis or atherosclerotic associated disease in a subject having or at risk of having atherosclerosis or atherosclerotic associated disease, wherein said agent or vaccine has the effect of reducing levels of bacteria which are elevated in the gut flora of the subject compared to the level in a control subject.
29. The antimicrobial agent or vaccine for use as in claim 28, wherein said use further comprises the step of detecting the presence of bacteria in the gut flora of said subject, and, where levels of said bacteria are elevated compared to the level in a control subject, administering an effective amount of an appropriate antimicrobial agent or vaccine.
30. The antimicrobial agent or vaccine for use as in claims 28 or 29, wherein said bacteria are *Collinsella* bacteria.

### References

1. Backhed F, Ley RE, Sonnenburg JL, Peterson DA, & Gordon JI (2005) Host-bacterial mutualism in the human intestine. Science 307(5717):1915-1920.
2. Cani PD, et al. (2007) Metabolic endotoxemia initiates obesity and insulin resistance. Diabetes 56(7):1761-1772.
3. Backhed F, et al. (2004) The gut microbiota as an environmental factor that regulates fat storage. Proc Natl Acad Sci USA 101(44):15718-15723.
4. Ley RE, Turnbaugh PJ, Klein S, & Gordon JI (2006) Microbial ecology: human gut microbes associated with obesity. Nature 444(7122):1022-1023.
5. Erridge C, Attina T, Spickett CM, & Webb DJ (2007) A high-fat meal induces low-grade endotoxemia: evidence of a novel mechanism of postprandial inflammation. Am J Clin Nutr 86(5):1286-1292.
6. Schertzer JD, et al. (2011) NODI Activators Link Innate Immunity to Insulin Resistance. Diabetes 60(9):2206-2215.
7. Koren O, et al. (2011) Human oral, gut, and plaque microbiota in patients with atherosclerosis. Proc Natl Acad Sci USA 108 Suppl 1:4592-4598.
8. Wang Z, et al. (2011) Gut flora metabolism of phosphatidylcholine promotes cardiovascular disease. Nature 472(7341):57-63.
9. Qin J, et al. (2010) A human gut microbial gene catalogue established by metagenomic sequencing. Nature 464(7285):59-65 .
10. Anonymous (2012) Structure, function and diversity of the healthy human microbiome. Nature 486(7402):207-214.
11. Greenblum S, Turnbaugh PJ, & Borenstein E (2012) Metagenomic systems biology of the human gut microbiome reveals topological shifts associated with obesity and inflammatory bowel disease. Proc Natl Acad Sci U S A 109(2):594-599.
12. Fagerberg B, et al. (2010) Differences in lesion severity and cellular composition between in vivo assessed upstream and downstream sides of human symptomatic carotid atherosclerotic plaques. J Vasc Res 47(3):221-230.
13. Christensen H & Boysen G (2004) C-reactive protein and white blood cell count increases in the first 24 hours after acute stroke. Cerebrovasc Dis 18(3):214-219.
14. Arumugam M, et al. (2011) Enterotypes of the human gut microbiome. Nature 473(7346):174-180.
15. Tap J, et al. (2009) Towards the human intestinal microbiota phylogenetic core. Environ Microbiol 11(10):2574-2584.
16. Wu GD, et al. (2011) Linking Long-Term Dietary Patterns with Gut Microbial Enterotypes. Science*.*
17. Zhu W, Lomsadze A, & Borodovsky M (2010) Ab initio gene identification in metagenomic sequences. Nucleic Acids Res 38(12):e132.
18. Oliveira AP, Patil KR, & Nielsen J (2008) Architecture of transcriptional regulatory circuits is knitted over the topology of bio-molecular interaction networks. BMC Syst Biol 2:17.
19. Patil KR & Nielsen J (2005) Uncovering transcriptional regulation of metabolism by using metabolic network topology. Proc Natl Acad Sci US A 102(8):2685- 2689.
20. Maslowski KM, et al. (2009) Regulation of inflammatory responses by gut microbiota and chemoattractant receptor GPR43. Nature 461(7268):1282-1286.
21. Clarke TB, et al. (2010) Recognition of peptidoglycan from the microbiota by Nod1 enhances systemic innate immunity. Nat Med 16(2):228-231.
22. Hansson GK (2005) Inflammation, atherosclerosis, and coronary artery disease. N Engl J Med 352(16):1685-1695.
23. Kardinaal AF, et al. (1993) Antioxidants in adipose tissue and risk of myocardial infarction: the EURAMIC Study. Lancet 342(8884):1379-1384.
24. Kohlmeier L, et al. (1997) Lycopene and myocardial infarction risk in the EURAMIC Study. Am J Epidemiol 146(8):618-626.
25. Hennekens CH, et al. (1996) Lack of effect of long-term supplementation with beta carotene on the incidence of malignant neoplasms and cardiovascular disease. N Engl J Med 334(18):1145-1149.
26. Kritchevsky SB (1999) beta-Carotene, carotenoids and the prevention of coronary heart disease. J Nutr 129(1):5-8.
27. Rissanen TH, et al. (2003) Serum lycopene concentrations and carotid atherosclerosis: the Kuopio Ischaemic Heart Disease Risk Factor Study. Am J Clin Nutr 77(1):133-138.
28. Sesso HD, Buring JE, Norkus EP, & Gaziano JM (2004) Plasma lycopene, other carotenoids, and retinol and the risk of cardiovascular disease in women. Am J Clin Nutr 79(1):47-53.
29. Bermudez OI, Ribaya-Mercado JD, Talegawkar SA, & Tucker KL (2005) Hispanic and non- Hispanic white elders from Massachusetts have different patterns of carotenoid intake and plasma concentrations. J Nutr 135(6):1496-1502.
30. Khaneja R, et al. (2010) Carotenoids found in Bacillus. Journal of Applied Microbiology 108(6):1889-1902.
31. Perez-Fons L, et al. (2011) Identification and the developmental formation of carotenoid pigments in the yellow/orange Bacillus spore-formers. Biochim Biophys Acta 1811(3):177-185.
32. Fagerberg B, Kellis D, Bergstrom G, & Behre CJ (2011) Adiponectin in relation to insulin sensitivity and insulin secretion in the development of type 2 diabetes: a prospective study in 64-year-old women. J Intern Med 269(6):636-643.
33. Schmidt C & Wikstrand J (2009) High apoB/apoA-I ratio is associated with increased progression rate of carotid artery intima-media thickness in clinically healthy 58-year-old men: experiences from very long-term follow-up in the AIR study. Atherosclerosis 205(1):284-289.
34. Mathiesen EB, Bonaa KH, & Joakimsen O (2001) Echolucent plaques are associated with high risk of ischemic cerebrovascular events in carotid stenosis: the tromso study. Circulation 103(17):2171-2175.
35. Prahl U, et al. (2010) Percentage white: a new feature for ultrasound classification of plaque echogenicity in carotid artery atherosclerosis. Ultrasound Med Biol 36(2):218-226.
36. Salonen A, et al. (2010) Comparative analysis of fecal DNA extraction methods with phylogenetic microarray: effective recovery of bacterial and archaeal DNA using mechanical cell lysis. J Microbiol Methods 81(2):127-134.
37. Cox MP, Peterson DA, & Biggs PJ (2010) SolexaQA: At-a-glance quality assessment of Illumina second-generation sequencing data. BMC Bioinformatics 11:485.
38. Langmead B, Trapnell C, Pop M, & Salzberg SL (2009) Ultrafast and memory-efficient alignment of short DNA sequences to the human genome. Genome Biol 10(3):R25.
39. Zerbino DR & Birney E (2008) Velvet: algorithms for de novo short read assembly using de Bruijn graphs. Genome Res 18(5):821-829.
40. Edgar RC (2010) Search and clustering orders of magnitude faster than BLAST. Bioinformatics 26(19):2460-2461.
41. Eddy SR (1998) Profile hidden Markov models. Bioinformatics 14(9):755-763.
42. Cantarel BL, et al. (2009) The Carbohydrate-Active EnZymes database (CAZy): an expert resource for Glycogenomics. Nucleic Acids Res 37(Database issue):D233-238.
43. Benjamini Y & Hochberg Y (1995) Controlling the False Discovery Rate - a Practical and Powerful Approach to Multiple Testing. J Roy Stat Soc B Met 57(1):289-300.
44. Dray S & Dufour AB (2007) The ade4 package: Implementing the duality diagram for ecologists. J Stat Softw 22(4):1-20.
45. Sowell AL, Huff DL, Yeager PR, Caudill SP, & Gunter EW (1994) Retinol, alpha-tocopherol, lutein/zeaxanthin, beta-cryptoxanthin, lycopene, alpha-carotene, trans-beta-carotene, and four retinyl esters in serum determined simultaneously by reversed-phase HPLC with multiwavelength detection. Clin Chem 40(3):411-416.

## Claims

1. β-carotene for use in treating or preventing atherosclerosis or atherosclerotic associated disease in a subject having or at risk of having atherosclerosis or atherosclerotic associated disease, wherein the β-carotene comprises a probiotic bacteria producing β-carotene.

2. The β-carotene for use according to claim 1, wherein said probiotic bacteria comprises a *Lactobacillus reuteri* strain producing β-carotene.

3. The β-carotene for use according to claim 1 or claim 2, wherein the probiotic bacteria are administered in the amount of 10³ to 10¹² CFU per day.
